(19) Europäisches Patentamt
European Patent Office
Office européen des brevets

(11) Veröffentlichungsnummer: **0 513 558 A1**

(12) **EUROPÄISCHE PATENTANMELDUNG**

(21) Anmeldenummer: **92106757.5**

(22) Anmeldetag: **21.04.92**

(51) Int. Cl.5: **C09B 23/10**, C09B 29/00, C07D 487/04

(30) Priorität: **08.05.91 DE 4114990**

(43) Veröffentlichungstag der Anmeldung: **19.11.92 Patentblatt 92/47**

(84) Benannte Vertragsstaaten: **CH DE FR GB IT LI**

(71) Anmelder: **BASF Aktiengesellschaft**
**Carl-Bosch-Strasse 38**
**W-6700 Ludwigshafen(DE)**

(72) Erfinder: **Guentner, Andreas, Dr.**
**Schubertstrasse 4**
**W-6804 Ilvesheim(DE)**
Erfinder: **Seybold, Guenther, Dr.**
**Friedrich-Ebert-Strasse 14**
**W-6708 Neuhofen(DE)**
Erfinder: **Wagenblast, Gerhard Dr.**
**Bachweg 8**
**W-6719 Weisenheim(DE)**

(54) **Tryptanthrinderivate.**

(57) Tryptanthrinderivate der Formel

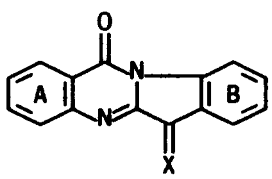

,

in der
die Ringe A und B benzoanelliert und/oder substituiert sein können und
X einen Rest, der sich von einem CH-aciden Heterocyclus, der gegebenenfalls einen weiteren Tryptanthrinrest aufweist, oder von einem Hydrazinoanthrachinon ableitet oder, wenn mindestens einer der Ringe A und B benzoanelliert und/oder durch Phthaloyl substituiert sind, auch Sauerstoff bedeutet,
sowie die Verwendung dieser Verbindungen als Farbmittel.

EP 0 513 558 A1

Die vorliegende Erfindung betrifft neue Tryptanthrinderivate der Formel I

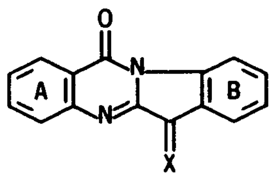

(I),

in der
die Ringe A und B gleich oder verschieden sind und unabhängig voneinander jeweils gegebenenfalls benzoanelliert sind und durch Halogen, $C_1$-$C_4$-Alkyl, $C_1$-$C_1$-Alkoxy, Carboxyl, $C_1$-$C_4$-Alkoxycarbonyl, Carbamoyl oder Hydroxysulfonyl ein- bis dreifach oder durch Phthaloyl einfach substituiert sein können und X einen Rest der Formel

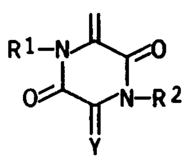

(IIa) ,

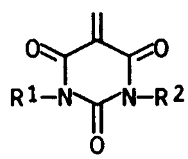

(IIb) ,

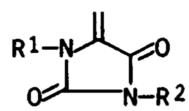

(IIc) ,

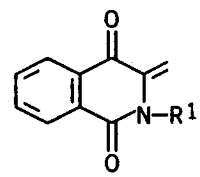

(IId) ,

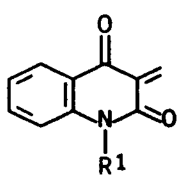

(IIe)

oder

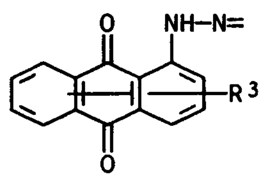

(IIf) ,

worin $R^1$ und $R^2$ gleich oder verschieden sind und unabhängig voneinander jeweils für Wasserstoff oder $C_1$-$C_4$-Alkyl, $R^3$ für Wasserstoff, $C_1$-$C_4$-Alkyl, Halogen oder $C_1$-$C_4$-Alkoxy und Y für Sauerstoff oder einen Rest der Formel

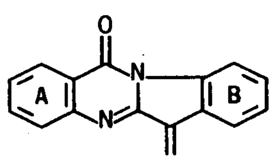

stehen, worin die Ringe A und B jeweils die obengenannte Bedeutung besitzen, oder wenn mindestens einer der Ringe A und B benzoanelliert und/oder durch Phthaloyl substituiert ist, auch Sauerstoff bedeutet, sowie die Verwendung dieser Verbindungen als Farbmittel.

In Tetrahedron Lett. Band 30, Seiten 2625 und 2626, 1977, sind Tryptanthrin der Formel

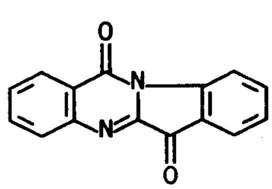

,

dort auch als Couropitin A bezeichnet, sowie einige seiner durch Chlor oder Brom substituierten Derivate

beschrieben.

Weiterhin sind aus Tetrahedron, Band 41, Seiten 2883 und 2884, 1985, die Kondensationsprodukte von Tryptanthrin mit Malodinitril und 3-Hydroxyindol bekannt.

J. Chem. Soc. Perkin Trans. I, Seiten 519 bis 527, 1987, beschreibt ein verdoppeltes Tryptanthrin. Schließlich ist aus dem DE-Patent Nr. 287 373 das Phenylhydrazon von Tryptanthrin bekannt.

Es hat sich jedoch gezeigt, daß diese Produkte bei ihrer Anwendung als Farbmittel noch Mängel aufweisen.

Aufgabe der vorliegenden Erfindung war es daher, neue Tryptanthrinderivate bereitzustellen, die über vorteilhafte anwendungstechnische Eigenschaften verfügen.

Demgemäß wurden die eingangs näher bezeichneten Tryptanthrinderivate der Formel I gefunden.

Alle in der obengenannten Formel I auftretenden ALkylgruppen können sowohl geradkettig als auch verzweigt sein.

Geeignete Substituenten für die Ringe A und B, wie auch die Reste $R^1$, $R^2$ und $R^3$ sind, z.B. Methyl, Ethyl, Propyl, Isopropyl, Butyl, Isobutyl oder sec-Butyl.

Geeignete Substituenten für die Ringe A und B, wie auch die Reste $R^3$, sind weiterhin z.B. Methoxy, Ethoxy, Propoxy, Isopropoxy, Butoxy, Fluor, Chlor oder Brom.

Geeignete Substituenten für die Ringe A und B sind weiterhin z.B. Methoxycarbonyl, Ethoxycarbonyl, Propoxycarbonyl, Isopropoxycarbonyl oder Butoxycarbonyl.

Bevorzugt sind Tryptanthrinderivate der Formel I, in der die Ringe A und B unabhängig voneinander gegebenenfalls benzoanelliert sind und durch Chlor oder Brom ein- oder zweifach oder durch Phthaloyl einfach substituiert sein können.

Weiterhin bevorzugt sind Tryptanthrinderivate der Formel I, in der X einen Rest der Formel IIa, IIc oder IIf bedeutet.

Besonders bevorzugt sind Tryptanthrinderivate der Formel I, in der X einen Rest der Formel

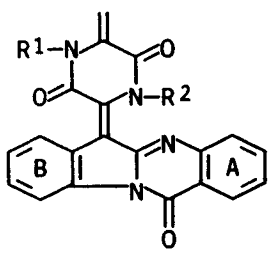 , 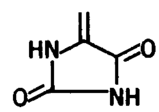 oder 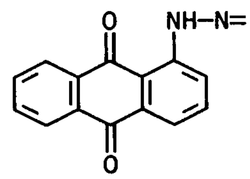

bedeutet, worin $R^1$ und $R^2$ unabhängig voneinander für Wasserstoff oder Methyl, insbesondere Wasserstoff, stehen und die Ringe A und B jeweils die obengenannte Bedeutung besitzen.

Die neuen Tryptanthrinderivate der Formel I können nach an sich bekannten Methoden erhalten werden.

Beispielsweise kann man ein Isatosäureanhydrid der Formel II

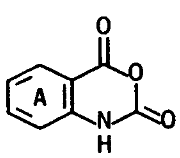 (II),

in der der Ring A die obengenannte Bedeutung besitzt, mit einem Isatin der Formel III

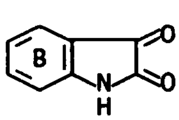 (III),

in der der Ring B die obengenannte Bedeutung besitzt, zur Reaktion bringen, wobei als Reaktionsprodukt ein Tryptanthrin der Formel Ia

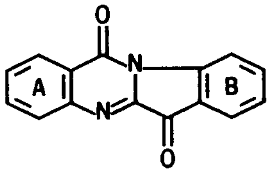

(Ia),

in der die Ringe A und B jeweils die obengenannte Bedeutung besitzen, entsteht.

Isatosäureanhydrid II und Isatin III werden dabei in der Regel in äquimolarem Verhältnis angewandt. Die Umsetzung kann z.B. in Pyridin als Verdünnungsmittel bei einer Temperatur von 95 bis 110 °C vorgenommen werden.

Diejenigen Tryptanthrine der Formel I, in der X eine andere Bedeutung als Sauerstoff besitzt, können durch Umsetzung der Tryptanthrine Ia mit einer Verbindung der Formel IV

$ZH_2$ (IV),

worin Z einen Rest der Formel IIa, IIb, IIc, IId, IIe oder IIf bedeutet, erhalten werden.

Wenn Z für einen Rest der Formel IIa, IIb, IIc, IId und IIe steht, ist es zweckmäßig, von solchen Verbindungen auszugehen, in denen die Reste $R^1$ und $R^2$ jeweils Acetyl bedeuten.

Die Kondensationsreaktion kann im allgemeinen mit gutem Erfolg in N,N-Dimethylformamid in Gegenwart einer Base, z.B. Triethylamin, oder in Acetanhydrid in Gegenwart von Alkaliacetat, z.B. Natriumacetat, bei einer Temperatur von 50 bis 150 °C vorgenommen werden. Das Molverhältnis Tryptanthrin Ia:Verbindung IV beträgt dabei in der Regel 0,8:1 bis 1,2:1.

Bei der Herstellung derjenigen Verbindungen der Formel I, in der X den Rest IIa bedeutet, worin Y für einen Rest der Formel

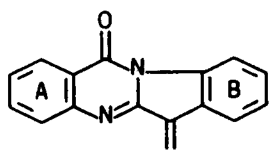

steht, beträgt das Molverhältnis Tryptanthrin:Piperazindion im allgemeinen 1:1 bis 2,2:1. (Als Piperazindion wird dabei die Verbindung der Formel IVa

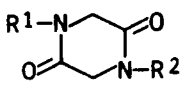

(IVa),

in der $R^1$ und $R^2$ jeweils die obengenannte Bedeutung besitzen, bezeichnet.)

Ähnliche Umsetzungen sind auch aus J. Heterocycl. Chem., Band 25, Seiten 591 bis 596, 1988, bekannt.

Diejenigen Verbindungen der Formel I, in der $R^1$ und $R^2$ für $C_1$-$C_4$-Alkyl steht, können durch Alkylierung der entsprechend unsubstituierten =NH-Verbindungen auf an sich bekanntem Wege erhalten werden.

Wenn Z in Formel IV den Rest der Formel IIf bedeutet, wird die Hydrazonbildung zweckmäßig in N,N-Dimethylformamid in Gegenwart von Eisessig bei einer Temperatur von 50 bis 120 °C vorgenommen. Die als Ausgangsverbindungen verwendeten 1-Hydrazinanthrachinone sind z.B. in Chem. Ber. Band 45, Seiten 2244 bis 2248, 1912, beschrieben.

Diejenigen Verbindungen der Formel I, in der die Ringe A und/oder B Halogen bedeutet, können auch durch aromatische Halogenierung der entsprechend unsubstituierten Tryptanthrinderivate der Formel I auf an sich bekanntem Wege erhalten werden.

Die neuen Tryptanthrinderivate können in vorteilhafter Weise als Farbmittel verwendet werden. Sie können dabei jeweils für sich allein oder auch in Mischung untereinander zur Anwendung kommen.

Sie eignen sich vorteilhaft als Pigmente zur Herstellung von Lacken. Weiterhin stellen sie wertvolle Zwischenprodukte für die Herstellung von Küpenfarbstoffen dar.

Die folgenden Beispiele sollen die Erfindung näher erläutern.

Beispiel 1

14,7 g Isatin und 19,8 g 4-Chlorisatosäureanhydrid wurden in 50 ml Pyridin für 6 Stunden unter Rückfluß erhitzt. Danach wurde das Reaktionsgemisch abgekühlt, der angefallene Niederschlag abgesaugt, mit Methanol gewaschen und getrocknet. Man erhielt 6,3 g (23 %) der Verbindung der Formel

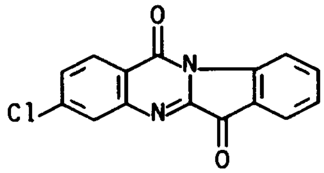

Analysenwerte
($C_{15}H_7N_2O_2Cl$): C 63,6 H 2,6 N 9,8 O 12,0 Cl 12,2
Schmelzpunkt: 282°C

In analoger Weise werden die in der folgenden Tabelle 1 aufgeführten Verbindungen erhalten.

Tabelle 1

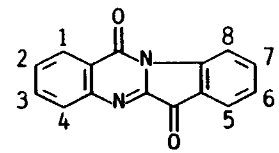

| Bsp. Nr. | Substituenten an Ringposition 1 | 2 | 3 | 4 | 5 | 6 | Ausbeute [%] | Fp. [°C] | Summenformel Analysenwerte |
|---|---|---|---|---|---|---|---|---|---|
| 2 | Cl | | | Cl | | | 46 | 290-330 | $C_{15}H_{5,9}N_2O_2Cl_{2,1}$ x 0·3 $H_2O$<br>C 55 H 2,1 N 8,7 O 11,4 Cl 22,9 |
| 3 | | Br | | Br | | | 31 | 269-286 | $C_{15}H_{5,7}N_2O_2Br_{2,3}$ x 2 $H_2O$<br>C 41,5 H 1,4 N 6,4 O 8,0 Br 41,9 |
| 4 | | Br | | | | | 26 | 322 | $C_{15}H_7N_2OBr$<br>C 54,8 H 2,0 N 8,5 O 10,4 Br 23,6 |
| 5 | | Br | (Br) | Br | | | 32 | 355-364 | $C_{15}H_{5,7}N_2O_2Br_{2,3}$ x 0,2 $H_2O$<br>C 41,2 H 1,7 N 6,4 O 7,9 Br 42,2 |
| 6 | | Br | (Br) | Br | Br | | 36 | 297-305 | $C_{15}H_{4,7}N_2O_2Br_{3,3}$ x 0,15 $H_2O$<br>C 34,9 H 1,0 N 5,3 O 6,6 Br 54,1 |
| 7 | | Br | Cl | Cl | | | 37 | 304-314 | $C_{15}H_5N_2O_2Cl_{2,15}Br_{0,85}$ x 0,15 $H_2O$<br>C 46,3 H 1,4 N 7,2 O 8,8 Cl 19,3 Br 17,3 |
| 8 | Cl | Cl | | Cl | | | 39 | 290-297 | $C_{15}H_5N_2O_2Cl_3$<br>C 51,1 H 1,5 N 8,0 O 9,5 Cl 29,8 |

Tabelle 1 (Fortsetzung)

| Bsp. Nr. | Substituenten an Ringposition 1 | 2 | 3 | 4 | 5 | 6 | Ausbeute [%] | Fp. [°C] | Summenformel Analysenwerte |
|---|---|---|---|---|---|---|---|---|---|
| 9 | | Cl | | Br | | | 38 | 268-276 | $C_{15}H_{6,49}N_2O_2Cl_{1,13}Br_{0,38}$ x 0,1 $H_2O$<br>C 56,2 H 2,1 N 8,7 O 10,4 Cl 12,5 Br 9,6 |
| 10 | | Cl | | Cl | | Br | 41 | 269-296 | $C_{15}H_{5,31}N_2O_2BrCl_{1,69}$ x 0,33 $H_2O$<br>C 45,7 H 1,5 N 7,0 O 9,7 Cl 15,2 Br 20,4 |
| 11 | | 2,3 | 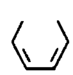 | | | | 30 | > 300 | $C_{19}H_{10}N_2O_2$<br>C 75,9 H 3,5 N 9,4 O 11,0 |
| 12 | | 3,4 | 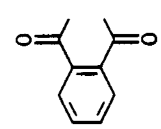 | | | | 40 | > 300 | $C_{23}H_{10}N_2O_4$ x 0,2 $H_2O$<br>C 71,9 H 2,8 N 7,1 O 17,5 |
| 13 | | 3,4 | 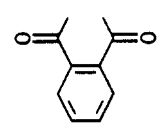 | | | Br | 34 | > 360 | $C_{23}H_9N_2O_4Br$<br>C 60,7 H 2,2 N 6,2 O 15,2 Br 15,1 |

Beispiel 14

6,2 g Tryptanthrin wurden in 30 ml N,N-Dimethylformamid (DMF) in Gegenwart von 1,7 g Triethylamin und 6,4 g Barbitursäure für 5 Stunden zum Sieden erhitzt. Nach dem Abkühlen wurde abgesaugt, mit DMF

und anschließend mit Methanol gewaschen und getrocknet. Es verblieben 0,8 g eines dunklen Pulvers der Formel

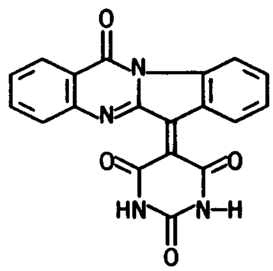

**Analysenwerte**
($C_{19}H_{10}N_4O_4$):
gef. C 63,2 H 4,3 N 13,4 O 18,5

Beispiel 15

35 g 4-Hydroxy-1-methylchinol-2-on wurden in 300 ml Acetanhydrid und 50 g Natriumacetat mit 50 g Tryptanthrin für 2 Stunden auf 110 °C erhitzt. Der gebildete Rückstand wurde abgesaugt, mit Methanol und anschließend mit Wasser gewaschen und getrocknet. Es verblieben 33,5 g eines rotbraunen Pulvers der Formel

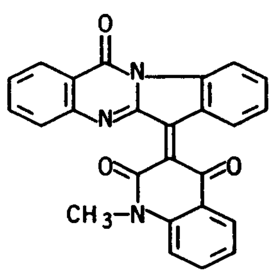

**Analysenwerte**
($C_{25}H_{15}N_3O_3$ x $H_2O$):
gef. C 72,0 H 3,6 N 9,3 O 14,9

Beispiel 16

2,5 g Tryptanthrin wurden in 50 ml DMF und 0,6 ml Triethylamin mit 1,8 g acetyliertem Hydantoin bei 50 °C 30 Minuten gerührt. Der Rückstand wurde abgesaugt, mit DMF und mit Methanol gewaschen und getrocknet. Man erhielt 1,8 g eines orangefarbenen Pulvers der Formel

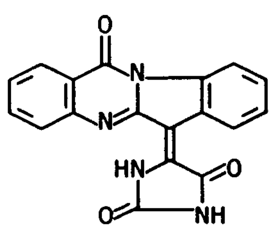

(monoacetyliert)

**Analysenwerte**
($C_{20}H_{12}N_4O_4$):
C 46,2 H 3,3 N 15,1 O 17,1

In analoger Weise wurden die in der folgenden Tabelle 2 aufgeführten Verbindungen erhalten.

Tabelle 2

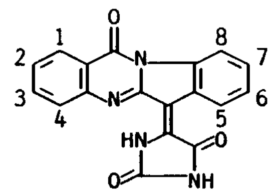

(monoacetyliert)

12

| Bsp. Nr. | Substituenten in Ringposition 2 | 3 | 6 | Farbe | Summenformel | Analysenwerte |
|---|---|---|---|---|---|---|
| 17 | | | Br | Orange | $C_{20}H_{11,12}N_4O_4Br_{0,88}$ x 0,2 $H_2O$ | C 53,6 H 2,7 N 12,5 O 14,9 Br 15,8 |
| 18 | 2,3 | 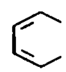 | | Rotbraun | $C_{24}H_{14}N_4O_4$ | C 67,7 H 3,5 N 13,0 O 15,3 |
| 19 | 3,4 | 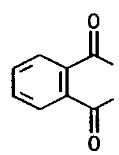 | | Braungelb | $C_{28}H_{14}N_4O_6$ | C 66,3 H 3,0 N 10,8 O 19,2 |
| 20 | 3,4, | 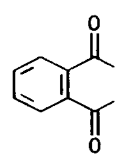 | Br | Braungelb | $C_{28}H_{13}N_3O_6Br$ | C 57,9 H 2,3 N 9,4 O 16,4 Br 12,9 (Cl 0,5) |

Beispiel 21

80 g Tryptanthrin wurden in 1000 ml DMF und 100 ml Eisessig bei 105 bis 110 °C gelöst. Danach wurden 130,3 g 1-Hydrazinoanthrachinon eingetragen. Das Reaktionsgemisch wurde 3 Stunden bei 110 °C

gerührt. Anschließend wurde bei 60 °C abgesaugt. Der Rückstand wurde mit DMF, dann mit Wasser und danach mit Methanol gewaschen und getrocknet.

Es verblieben 59 g eines roten Pulvers der Formel

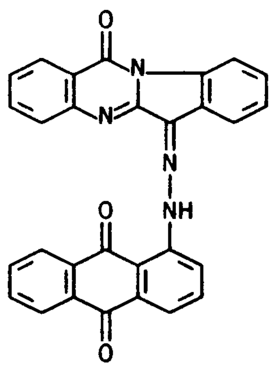

Analysenwerte ($C_{29}H_{16}N_4O_3$): C 74,3 H 3,5 N 11,7 O 10,5

In analoger Weise wurden die in der folgenden Tabelle 3 aufgeführten Verbindungen erhalten.

Tabelle 3

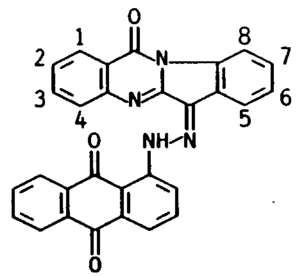

| Bsp. Nr. | Substituenten in Ringposition 1 | 2 | 3 | 4 | 5 | 6 | Farbe | Summenformel Analysenwerte |
|---|---|---|---|---|---|---|---|---|
| 22 | | Cl | | | | | Rot | $C_{29}H_{15}N_4O_3Cl$<br>C 68,9 H 3,1 N 11,3 O 10,3 Cl 6,5 |
| 23 | | | | | | Br | Rot | $C_{29}H_{15}N_4O_3Br$<br>C 63,2 H 2,8 N 10,1 O 9,1 Br 13,6 |
| 24 | | Cl | | (Cl) | | | Rot | $C_{29}H_{14,3}N_4O_3Cl_{1,7}$ x 0,15 $H_2O$<br>C 66,1 H 2,8 N 10,5 O 9,6 Cl 11,2 |
| 25 | Cl | | | | | | Rot | $C_{29}H_{15}N_4O_3Cl$<br>C 69,1 H 3,1 N 11,3 O 10,0 CFl 6,9 |
| 26 | | Br | | Br | | | Rot | $C_{29}H_{13,8}N_4O_3Br_{2,2}$ x 0,4 $H_2O$<br>C 53,8 H 2,3 N 8,7 O 8,4 Br 26,9 |

Tabelle 3 (Fortsetzung)

| Bsp. Nr. | Substituenten in Ringposition 1 | 2 | 3 | 4 | 5 | 6 | Farbe | Summenformel Analysenwerte |
|---|---|---|---|---|---|---|---|---|
| 27 | | Br | | | | | Rot | $C_{29}H_{15}N_4O_3Br$<br>C 63,4 H 2,7 N 10,1 O 9,0 Br 14,1 |
| 28 | | Br | (Br) | Br | | | Rot | $C_{29}H_{14}N_4O_3Br_{2,4} \times 0,2\ H_2O$<br>C 52,4 H 2,2 N 8,5 O 7,7 Br 28,6 |
| 29 | | Br | Br | Br | | Br | Braun | $C_{29}H_{12}N_4O_3Br_4$<br>C 45,2 H 1,6 N 7,6 O 7,2 Br 39,0 |
| 30 | | Br | Cl | Cl | | | Braun | $C_{29}H_{13}N_4O_3BrCl_2$<br>C 56,0 H 2,2 N 9,1 O 8,9 Br 11 Cl 12,4 |
| 31 | Cl | Cl | | Cl | | | Braunrot | $C_{29}H_{13}N_4O_3Cl_3$<br>C 60,1 H 2,4 N 9,8 O 9,1 Cl 18,3 |
| 32 | | Cl | | (Br) | | | Rot | $C_{29}H_{14,49}N_4O_3Br_{0,39}Cl_{1,12} \times 0,3\ H_2O$<br>C 63,9 H 2,8 N 10,1 O 9,6 Br 5,8 Cl 7,3 |
| 33 | | Cl | | Cl | | Br | Rot | $C_{29}H_{13}N_4O_3BrCl_2$<br>C 57,0 H 2,3 N 9,1 O 8,7 Br 12,1 Cl 10,1 |

Tabelle 3 (Fortsetzung)

| Bsp. Nr. | Substituenten in Ringposition 1 | 2 | 3 | 4 | 5 | 6 | Farbe | Summenformel Analysenwerte |
|---|---|---|---|---|---|---|---|---|
| 34 | | 2,3 | 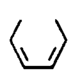 | | | | Dunkel-braun | $C_{33}H_{18}N_4O_3$ x 0,2 $H_2O$<br>C 75,4 H 3,5 N 10,6 O 9,6 |
| 35 | | 3,4 | 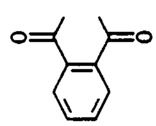 | | | | Dunkel-braun | $C_{37}H_{18}N_4O_5$<br>C 73,1 H 3,1 N 10,0 O 13,8 |
| 36 | | 3,4 | 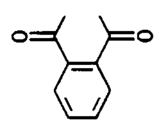 | | | Br | Dunkel-braun | $C_{37}H_{17}N_4O_5Br$<br>C 64,8 H 2,5 N 7,4 O 13,9 Br 10,8 |

Beispiel 37

7,44 g Tryptanthrin und 3 g N,N-Diacetylpiperazin-2,5-dion wurden in 120 ml DMF in Gegenwart von 1,5 g Triethylamin für 3 Stunden unter Rückfluß erhitzt. Danach wurde der verbleibende Rückstand abgesaugt und mit DMF und Methanol gewaschen. Nach dem Trocknen verblieben 8,2 g eines dunklen

Pulvers der Formel

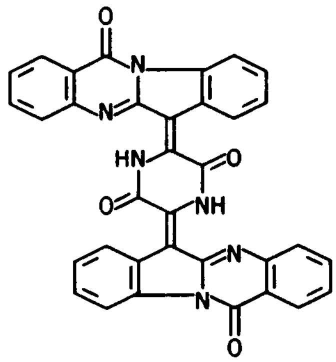

Analysenwerte ($C_{34}H_{18}N_6O_4$): C 70,4 H 3,3 N 14,5 O 11,8

In analoger Weise wurden die in der folgenden Tabelle 4 aufgeführten Verbindungen erhalten.

Tabelle 4

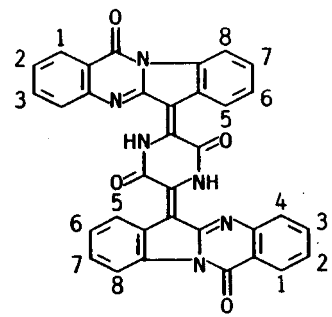

| Bsp. Nr. | Substituenten in Ringposition 1 | 2 | 3 | 4 | 5 | 6 | Farbe | Summenformel Analysenwerte |
|---|---|---|---|---|---|---|---|---|
| 38 | | Cl | | | | | Trüb-violett | $C_{34}H_{16}N_6O_4Cl_2$ C 61,6 H 2,8 N 12,7 O 12,4 Cl 10,2 |
| 39 | | | | | | Br | Grau | $C_{34}H_{16}N_6O_4Br_2$ C 56,3 H 2,5 N 11,7 O 9,9 Br 19,4 |
| 40 | | Cl | | Cl | | | Trüb-violett | $C_{34}H_{14}N_6O_4Cl_4$ C 58,0 H 2,4 N 12,1 O 10,5 Cl 16,9 |
| 41 | | | Cl | | | | Trüb-braun | $C_{34}H_{16}N_6O_4Cl_2$ C 62,8 H 2,7 N 12,9 O 11,1 Cl 10,5 |
| 42 | Cl | | | | | | Trüb-violett | $C_{34}H_{16}N_6O_4Cl_2$ C 62,6 H 28 N 13,0 O 11,3 Cl 10,4 |

Tabelle 4 (Fortsetzung)

| Bsp. Nr. | Substituenten in Ringposition 1 | 2 | 3 | 4 | 5 | 6 | Farbe | Summenformel Analysenwerte |
|---|---|---|---|---|---|---|---|---|
| 43 | Cl | | | | | | Grau | $C_{34}H_{14}N_6O_4Cl_4$<br>C 57,3 H 2,6 N 11,8 O 10,4 Cl 18,2 |
| 44 | | Cl | Cl | Cl | | | Trüb-blau | $C_{34}H_{12}N_6O_4Cl_6$<br>C 51,3 H 1,7 N 10,5 O 9,4 Cl 26,9 |
| 45 | | Br | | Br | | | Trüb-violett | $C_{34}H_{14}N_6O_4Br_4$<br>C 43,8 H 1,7 N 9,0 O 8,0 Br 37,3 |
| 46 | | $(SO_3)Na)$ | | | | | Trüb-blau | $C_{34}H_{16,3}N_6O_{14,2}S_{1,7}Na_{1,7} \times H_2O$<br>C 46,8 H 4,2 N 9,8 O 27,7 S 6,1 Na 5,1 |
| 47 | | Br | | | | | Trüb-blau | $C_{34}H_{16}N_6O_4Br_2$<br>C 55,4 H 2,6 N 11,5 O 10,1 Br 20,2 |
| 48 | | Br | (Br) | Br | | (Br) | Grau | $C_{34}H_{11,5}N_6O_4Br_{6,5} \times 0,75\ H_2O$<br>C 37,1 H 1,2 N 7,6 O 6,9 Br 47,2 |
| 49 | | Br | Br | Br | | | Grau-violett | $C_{34}H_{12}N_6O_4Br_2Q_4$<br>C 47,7 H 1,7 N 9,7 O 8,7 Br 15,4 Cl 17,1 |

Tabelle 4 (Fortsetzung)

| Bsp. Nr. | Substituenten in Ringposition 1 | 2 | 3 | 4 | 5 | 6 | Farbe | Summenformel Analysenwerte |
|---|---|---|---|---|---|---|---|---|
| 50 | Cl | Cl | | Cl | | | Grau-violett | $C_{34}H_{12}N_6O_4Cl_6$<br>C 52,1 H 1,8 N 10,5 O 9,2 Cl 26,0 |
| 51 | | Cl | | (Br) | | | Grau | $C_{34}H_{14,9}N_6O_4Br_{0,9}Cl_{2,2}$ x 0,4 $H_2O$<br>C 56 H 2,3 N 11,3 O 9,6 Br 9,7 Cl 10,8 |
| 52 | | Cl | | (Cl) | | Br | Braun-grau | $C_{34}H_{13,2}N_6O_4Br_{1,85}Cl_{2,95}$ x 1,3 $H_2O$<br>C 48 H 2,3 N 10 O 9,9 Br 17,3 Cl 12,2 |
| 53 | | 2,3 | 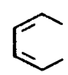 | | | | Violett | $C_{42}H_{22}N_6O_4$ x 0,7 $H_2O$<br>C 73,3 H 3,4 N 12,2 O 10,9 |
| 54 | | 3,4 | 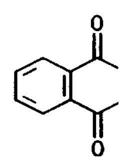 | | | | Violett | $C_{50}H_{22}N_6O_8$ x $H_2O$<br>C 70,3 H 2,9 N 9,9 O 16,4 |
| 55 | | 3,4 | 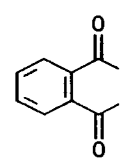 | | | Br | Schwarz | $C_{50}H_{20}N_6O_8Br_2$<br>C 60,5 H 2,0 N 8,1 O 4,6 Br 13,9 |

Beispiel 56

11,5 g der Verbindung aus Beispiel 37 wurde in 70 ml DMF und 2,9 g Natriumhydrid vorgelegt. In die resultierende blaue Lösung wurden bei Raumtemperatur 6 g Methyliodid getropft. Man rührte 3 Stunden bei

Raumtemperatur, saugte ab, wusch und mit DMF, Methanol und heißem Wasser nach und trocknete. Man erhielt 7,7 g eines dunklen Pulvers der Formel

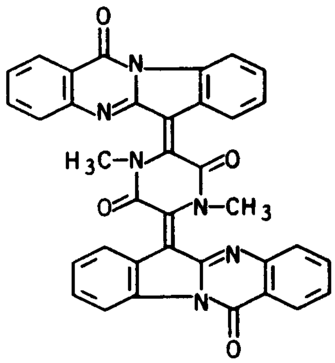

Fp.: > 350°C

Analysenwerte

($C_{36}H_{22}N_6O_4$ x 0,6 $H_2O$):

C 70,3 H 3,6 N 14,0 O 12,0

**Patentansprüche**

**1.** Tryptanthrinderivate der Formel I

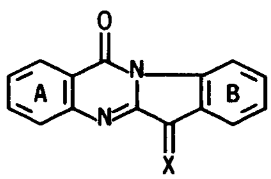

(I),

in der

die Ringe A und B gleich oder verschieden sind und unabhängig voneinander jeweils gegebenenfalls benzoanelliert sind und durch Halogen, $C_1$-$C_4$-Alkyl, $C_1$-$C_1$-Alkoxy, Carboxyl, $C_1$-$C_4$-Alkoxycarbonyl, Carbamoyl oder Hydroxysulfonyl ein- bis dreifach oder durch Phthaloyl einfach substituiert sein können und X einen Rest der Formel

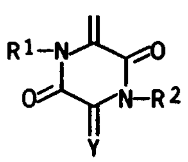

(IIa),

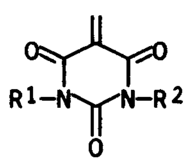

(IIb),

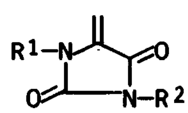

(IIc),

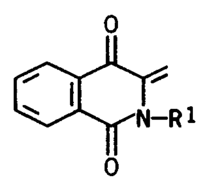

(IId),

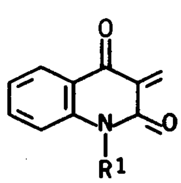

(IIe)

oder

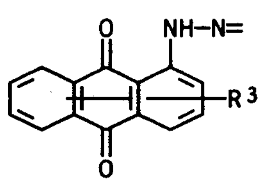

(IIf),

worin $R^1$ und $R^2$ gleich oder verschieden sind und unabhängig voneinander jeweils für Wasserstoff oder $C_1$-$C_4$-Alkyl, $R^3$ für Wasserstoff, $C_1$-$C_4$-Alkyl, Halogen oder $C_1$-$C_4$-Alkoxy und Y für Sauerstoff oder einen Rest der Formel

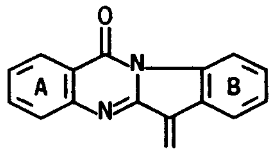

stehen, worin die Ringe A und B jeweils die obengenannte Bedeutung besitzen, oder wenn mindestens einer der Ringe A und B benzoanelliert und/oder durch Phthaloyl substituiert ist, auch Sauerstoff bedeutet.

2. Tryptanthrinderivate nach Anspruch 1, dadurch gekennzeichnet, daß die Ringe A und B unabhängig voneinander gegebenenfalls benzoanelliert sind und durch Chlor oder Brom ein- oder zweifach oder durch Phthaloyl einfach substituiert sein können.

3. Tryptanthrinderivate nach Anspruch 1, dadurch gekennzeichnet, daß X einen Rest der Formel IIa, IIc oder IIf bedeutet.

4. Tryptanthrinderivate nach Anspruch 1, dadurch gekennzeichnet, daß X einen Rest der Formel

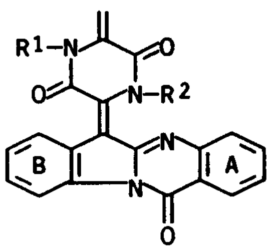 , 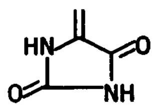 oder 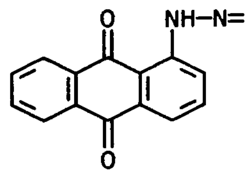

bedeutet, worin $R^1$ und $R^2$ unabhängig voneinander für Wasserstoff oder Methyl stehen und die Ringe A und B jeweils die in Anspruch 1 genannte Bedeutung besitzen.

5. Verwendung der Tryptanthrinderivate gemäß Anspruch 1 als Farbmittel.

Europäisches Patentamt

# EUROPÄISCHER RECHERCHENBERICHT

Nummer der Anmeldung

EP 92 10 6757

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| D,A | TETRAHEDRON<br>Bd. 41, Nr. 14, 1985, GB<br>Seiten 2883 - 2884; 'STRUCTURE DETERMINATION OF CANDIDINE'<br>* der ganze Artikel *<br>--- | 1 | C09B23/10<br>C09B29/00<br>C07D487/04 |
| D,A | JOURNAL OF THE CHEMICAL SOCIETY, PERKIN TRANSACTIONS 1.<br>Nr. 3, März 1987, LETCHWORTH GB<br>Seiten 519 - 527;<br>J. BERGMAN; U. TILSTAM: 'Reduction and Stereochemical Studies through N.M.R. and X-Ray Technics of Indoloquinazolins'<br>* Formeln 2,40,41 *<br>--- | 1 | |
| A | DYES AND PIGMENTS.<br>Bd. 5, Nr. 5, 1984, BARKING GB<br>Seiten 189 - 207;<br>M. ROLF; R. NEEFF: 'Neue, hochechte organische Pigmente'<br>* Formeln auf den Seiten 191,198 *<br>--- | 1,4 | **RECHERCHIERTE SACHGEBIETE (Int. Cl.5 )** |
| D,A | DE-C-287 373 (BASF)<br>* das ganze Dokument *<br>--- | 1,5 | C09B<br>C07D |
| A | EP-A-0 151 393 (BAYER)<br>* Formeln *<br>----- | 1 | |

Der vorliegende Recherchenbericht wurde für alle Patentansprüche erstellt

| Recherchenort | Abschlußdatum der Recherche | Prüfer |
|---|---|---|
| DEN HAAG | 26 JUNI 1992 | KETTERER M. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)